# EUROPEAN PATENT SPECIFICATION

(11) **EP 2 844 334 B1**
(45) Date of publication and mention of the grant of the patent: **16.01.2019**
(21) Application number: 13711196.9
(22) Date of filing: 01.03.2013
(51) Int. Cl.: A61N 1/05

(54) **PACING LEADS WITH ULTRATHIN ISOLATION LAYER BY ATOMIC LAYER DEPOSITION**
SCHRITTMACHERLEITUNGEN MIT ULTRADÜNNER ISOLIERSCHICHT MITTELS ATOMLAGENABSCHEIDUNG
SONDES DE STIMULATION AVEC COUCHE ISOLANTE ULTRAMINCE PAR DÉPÔT DE COUCHE ATOMIQUE

(30) Priority: 02.05.2012 US 201261641460 P
(43) Date of publication of application: 11.03.2015
(73) Proprietor: Cardiac Pacemakers, Inc., St. Paul, Minnesota 55112 (US)
(72) Inventor: WEBER, Jan, 6228 GJ Maastricht (NL); BYRON, Mary M., Roseville, Minnesota 55113 (US)
(74) Representative: Pfenning, Meinig & Partner mbB
(86) International application number: PCT/US2013/028692
(87) International publication number: WO 2013/165544

(56) References cited:
- EP-A1- 0 585 553
- WO-A1-2009/134901
- WO-A1-2011/031316

## Description

### TECHNICAL FIELD

The present invention relates to medical devices and in particular, medical devices having an increased wear resistance. Methods of forming such medical devices are also discussed.

### BACKGROUND

Various polymer coated medical devices are known which are configured for implantation or insertion into a body of a patient.

Such devices have attendant medical requirements. For example the strength of the polymer coating is an important parameter in certain applications. Insulating properties may also be important.

EP0585553A1 relates to a multipolar electrode lead for a medical apparatus devised to deliver stimulation pulses to living tissue. Said electrode lead comprises an insulating sheath in which at least a first conductor and a second conductor are coiled. At least the first conductor is covered with an insulating coating to electrically insulate the conductors from one another. The insulated coating on the first conductor is highly resistant and abrasion-resistant.

EP0585553A1 does not disclose an implantable medical lead comprising at least one ceramic layer, wherein each ceramic layer is less than or equal to about 30 nanometers thick.

### SUMMARY

Disclosed herein are various embodiments of a medical device, as well as methods for making such medical devices.

In Example 1, an implantable medical lead includes a substrate having a surface, and a nanolaminate disposed on the surface of the substrate. The nanolaminate includes at least one ceramic layer and at least one polymer layer. Each ceramic layer is less than or equal to about 30 nanometers thick.

In Example 2, the implantable medical lead of claim 1, wherein a polymer layer is disposed between a first ceramic layer and a second ceramic layer.

In Example 3, the implantable medical lead of according to Example 1 or Example 2, wherein the ceramic layers and the polymer layers are arranged in an alternating pattern.

In Example 4, the implantable medical lead according to any of Examples 1-3, wherein the polymer layers are less than about 100 nanometers thick.

In Example 5, the implantable medical lead according to any of Examples 1-4, wherein the ceramic layer includes at least one component selected from the group consisting of aluminum oxide and titanium oxide.

In Example 6, the implantable medical lead according to any of Examples 1-5, wherein the polymer layer includes at least one member selected from the group consisting of polyamides and polyimides.

In Example 7, the implantable medical lead according to any of Examples 1-6, wherein the implantable medical lead comprises a tubular body having an outer surface and an inner surface, and wherein the nanolaminate is disposed on the outer surface.

In Example 8, the implantable medical lead according to any of Examples 1-7, wherein the implantable medical lead comprises a tubular body having an outer surface and an inner surface, and wherein the nanolaminate is disposed on the inner surface.

In Example 9, the implantable medical lead according to any of Examples 1-8 and further comprising at least one layer comprising a ceramic material and a polymer material.

In Example 10, a method of forming a nanolaminate on an implantable medical lead includes depositing a ceramic material on the implantable medical lead to form a ceramic layer having a thickness less than or equal to about 30 nanometers, and depositing a polymer material on the ceramic layer to form a polymer layer.

In Example 11, the method according to Example 10 and further comprising depositing a second ceramic material on the polymer layer to form a second ceramic layer.

In Example 12, the method according to Example 10 or Example 11 wherein the step of depositing a ceramic material includes depositing a ceramic material by atomic layer deposition (ALD).

In Example 13, the method according to any of Examples 10-12, wherein the step of depositing a ceramic material includes depositing a ceramic material by a sol gel process.

In Example 14, the method according to any of Examples 10-13, wherein the step of depositing a ceramic material includes depositing a ceramic material by liquid-source misted chemical deposition (LSMCD).

In Example 15, an implantable medical lead includes a substrate extending from a distal to a proximal end of the medical lead. The substrate includes at least one polymer material having particles of at least one nanoclay or nanodiamond material dispersed throughout the polymer material.

In Example 16, the implantable medical lead according to Example 15, wherein the nanoclay or nanodiamond material is homogenously dispersed throughout the polymer material.

In Example 17, the implantable medical lead according to Example 15 or Example 16 and further including a ceramic material dispersed throughout the polymer material.

In Example 18, the implantable medical lead according to any of Examples 15-17 wherein the polymer material includes at least one member selected from the group consisting of polyamide polymers, polyimide polymers, polyurethanes and silicones.

In Example 19, the implantable medical lead according to any of Examples 15-18, wherein the nanodiamond material includes diamond particles having a crystal size between about 1 nm and about 100 nm.

In Example 20, the implantable medical lead according to any of Examples 15-18 and further including a ceramic layer on the substrate.

While multiple embodiments are disclosed, still other embodiments of the present invention will become apparent to those skilled in the art from the following detailed description, which shows and describes illustrative embodiments of the invention. Accordingly, the drawings and detailed description are to be regarded as illustrative in nature and not restrictive.

### BRIEF DESCRIPTION OF THE DRAWINGS

FIG. 1 illustrates an exemplary embodiment of an implantable device.
FIG. 2 illustrates a cross-sectional view of an embodiment of an implantable lead including a nanolaminate.
FIG. 3 illustrates a cross-sectional view of an embodiment of an implantable lead including a nanolaminate between conductors and polymer insulation.
FIG. 4 illustrates a cross-sectional view of an embodiment of an implantable lead having a nanolaminate applied to an outer surface.
FIG. 5 illustrates a cross-sectional view of an embodiment of an implantable lead having a nanolaminate including a ceramic layer, a polymer layer and a hybrid layer.
FIG. 6 illustrates a cross-sectional view of an embodiment of a polymer material containing a ceramic material.

It is noted that the figures are not to scale in order to show sufficient detail.

While the invention is amenable to various modifications and alternative forms, specific embodiments have been shown by way of example in the drawings and are described in detail below. The intention, however, is not to limit the invention to the particular embodiments described. On the contrary, the invention is intended to cover all modifications, equivalents, and alternatives falling within the scope of the invention as defined by the appended claims.

### DETAILED DESCRIPTION

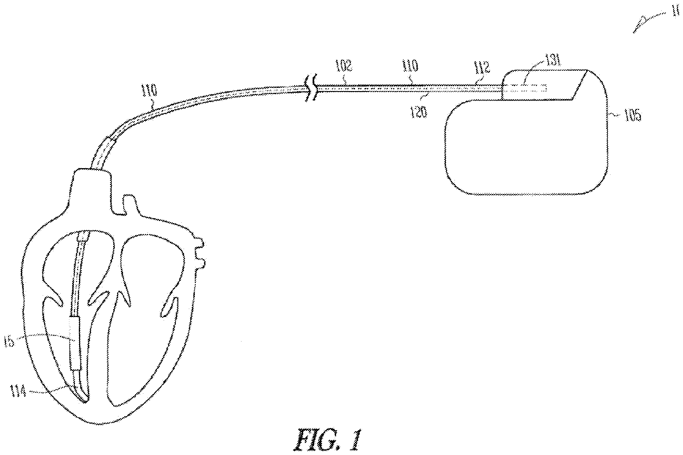

FIG 1 illustrates an implantable device 100, such as a lead 102 for use with a pulse generator 105. Implantable device 100 includes a lead body 110, and at least one elongate conductor 120 contained within the lead body 110. The lead body 110 extends from a proximal end 112 to a distal end 114. The proximal end 112 of the lead 102 is electrically coupled with the pulse generator 105, for example, with a terminal pin 131.

The implantable device 100 generically represents, but is not limited to, cardiac function management (referred to as "CFM") systems such as pacers, cardioverters/defibrillators, pacers/defibrillators, biventricular or other multi-site resynchronization or coordination devices such as cardiac resynchronization therapy (referred to as "CRT") devices, sensing instruments, drug delivery systems, neurostimulation devices, or organ stimulation devices. Alternatively, the lead 102 could be utilized for neuro-stimulation or other body implantable applications.

The pulse generator 105 includes a source of power as well as an electronic circuitry portion. In one example, the electronic circuitry includes microprocessors to provide processing, evaluation, and to determine and deliver electrical shocks or pulses of different energy levels and timing for neurostimulators or ventricular defibrillation, cardioversion, or pacing of heart in response to sensed cardiac arrhythmia including fibrillation, cardiac resynchronization, tachycardia, or bradycardia. In another example, the pulse generator 105 is a battery-powered device that senses intrinsic signals of the heart and generates a series of timed electrical discharges.

The implantable device 100 further includes one or more electrodes 115. The one or more electrodes 115 are each electrically coupled with the at least one conductor 120. The electrode 115 allows for electrical signals to be delivered to the tissue from the pulse generator 105. The implantable device 100 can further include features to allow the lead body 110 to be fixated within a patient. For example, the lead body 110 can include passive fixation features, such as one or more tines. In another option, the lead body 110 can include an active fixation assembly, such as a fixation helix.

The lead body 110 isolates electrically conductive components within the lead body 110 from surrounding tissues of the patient. In some embodiments, the components may rub or wear on the inner surface of the lead body 110. Overtime, this may result in failure of the isolation, which may result in short circuiting. In some embodiments described herein, the lead body 110 has an enhanced wear resistance. In other embodiments, the lead body has a redundant insulation system to reduce the risk of isolation failure.

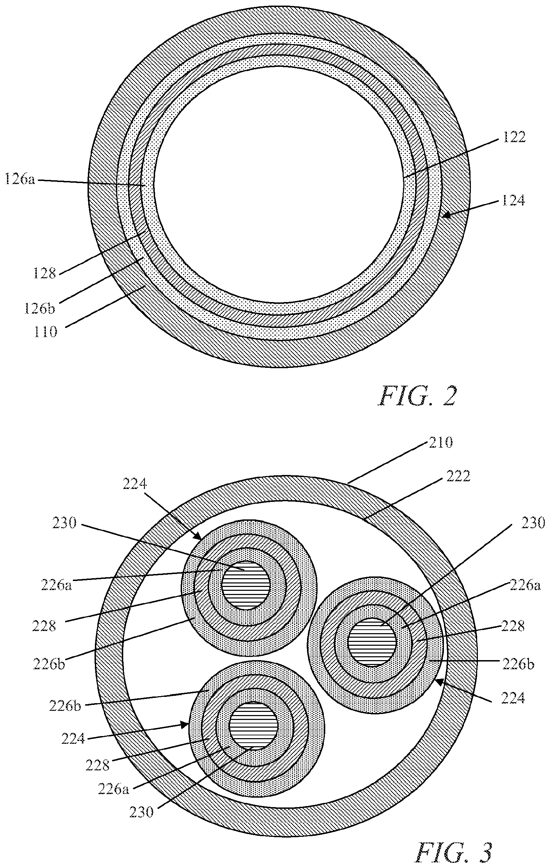

FIG. 2 illustrates a cross-sectional view of an embodiment of a lead body 110 with a lumen 122 which axially extends through the lead body 110, according to the present disclosure. A nanolaminate 124 is on the inner surface of the lead body 110 and comprises ceramic layers 126a and 126b (referred to collectively as ceramic layers 126) and a polymer layer 128. As shown, in some embodiments, the ceramic layers 126a and 126b and the polymer layer 128 can be arranged in an alternating pattern, and other patterns are also possible.

The nanolaminate 124 covers all or at least a portion of the inner surface of the lead body 110. For example, the nanolaminate 124 can extend from a distal end to a proximal end of the lead body 110.

Ceramic layers 126 include an inorganic or ceramic material, such as aluminum oxide, titanium oxide, bismuth oxide, vanadium oxide, hafnium oxide, zirconium oxide, silicone oxide, and zinc oxide, among others. As discussed herein, the lead body 110 can include multiple layers of the ceramic layers 126, for example, ceramic layers 126a and 126b. Each discrete ceramic layer 126 may have the same composition. In other embodiments, the ceramic layers 126 can have different compositions.

As described herein, the ceramic layers 126 may be formed by a self-limiting reaction process which provides control over the thickness of the ceramic layers 126. In one example, the ceramic layers 126 are formed by atomic layer deposition (ALD). Atomic layer deposition is a coating technique based on chemical vapor deposition (CVD). Atomic layer deposition is a binary chemical reaction that is split into two half-reactions in order to achieve a precise control over the film thickness. In some embodiments, the ceramic layers 126 can have a thickness equal to or less than about 30 nanometers (nm). In some embodiments, the ceramic layers 126 can have a thickness that is less than about 20 nm and in still further embodiments less than about 10 nm. In some embodiments, the ceramic layers 126 can have a thickness from about 1 nm to about 20 nm or from about 5 nm to about 20 nm.

As discussed briefly herein, a polymer layer 128 can be positioned between the ceramic layers 126. The polymer layer 128 includes a polymer material. Suitable polymer materials include but are not limited to polyamide polymers and polyimide polymers. Further suitable polymer materials include polyurethanes and silicones. In embodiments including more than one polymer layer 128, the polymer layers 128 may have the same or different compositions.

The polymer layer 128 can have a thickness on the nanometer scale. For example, in some embodiments, the polymer layer 128 can be between about 5 nm and about 100 nm.

Polymer materials typically have a relatively higher elasticity than ceramic materials. Ceramic materials can have a higher wear resistance and better insulating properties compared to polymer materials. It has been found that the ceramic layers 126 having a thickness greater than 30 nm may experience cracking and/or delamination, particularly at high strain points following deformation. Separating adjacent ceramic layers 126 with the polymer layers 128 enables the overall thickness of the nanolaminate 124 to be greater than 30 nm while maintaining individual ceramic layers 126 equal to or less than 30 nm thick so that the ceramic layers have an increased resistance to cracking and delamination. Nanolaminate 124 has a higher wear resistance and better insulating properties than a nanolaminate including only a polymer material. Further, the risk of delamination or cracking of the ceramic material is decreased because the individual ceramic layers 126 are equal to or less than 30 nm thick. For example, lead body 110 having nanolaminate 124 may be more resistant to wear caused by components rubbing on the inner surface of lead body 110.

Embodiments of the present disclosure can include a nanolaminate 124 that has various combinations of the ceramic layers 126 and the polymer layers 128 arranged in an alternating pattern. In one example, one ceramic layer 126 can be positioned immediately adjacent to the lumen 122 in order to provide enhanced wear resistance for the inner surface of the lead body 110. For example, enhanced wear resistance is experienced when the ceramic layer 126 has a higher wear resistance than the lead body 110. Additionally, the ceramic layer 126 can have a higher wear resistance than the polymer layer 128.

The polymer material of the polymer layer 128 may intertwine with the ceramic material of ceramic layer 126 at the interface of the ceramic layer 126 and the polymer layer 128. For example, the nanolaminate 124 may include an interface region between the ceramic layer 126 and the polymer layer 128 that includes the ceramic material and the polymer material.

The dielectric and mechanical properties of the nanolaminate 124 can be tailored by adjusting the thicknesses and/or composition of the ceramic layers 126 and the polymer layer 128. For example, the elasticity of the nanolaminate 124 can increase with increasing polymer content. In one embodiment, the polymer content of the nanolaminate 124 can increase with increasing the thickness of the polymer layers 128. In another embodiment, the polymer content of the nanolaminate 124 can increase with increasing the number of polymer layers 128. In one example, the ceramic layers 126 can include tantalum oxide and the polymer layers 128 can include a polyimide polymer.

The nanolaminate 124 can also have insulation properties. In embodiments in which the nanolaminate 124 is on a lead body 110, nanolaminate 124 can insulate the conductor 102. In some embodiments, the multiple ceramic layers 126 serve as a redundant insulation system that insulate or isolate electrical components within lead body 110 from a surrounding environment, such as tissues. In the event that one ceramic layer 126 fails or develops a weakness, the remaining ceramic layers 126 may provide sufficient insulation to protect the surrounding environment from the electrical component.

The nanolaminate 124 can be formed by various manufacturing processes. The ceramic layers 126 may be formed by a self-limiting reaction process which provides control over the thickness of the ceramic layers 126. In one example, the ceramic layers 126 are formed by atomic layer deposition (ALD). As described herein, atomic layer deposition is a coating technique based on chemical vapor deposition (CVD). Atomic layer deposition is a binary chemical reaction that is split into two half-reactions in order to achieve a precise control over the film thickness. The two half-reactions together may be referred to as a reaction cycle.

Each half-step is self limiting as the pre-cursor only reacts with available sites on the surface. In the first half-reaction, a first precursor species in vapor phase is fed into a reaction chamber and is chemisorbed onto the surface of the substrate (e.g., the inner surface of the lead body 110) by reacting with reactive sites. Chemisorpotion of the first precursor species proceeds until all free surface species on the substrate have reacted, at which time the reaction self-terminates, resulting in a monolayer. Once all free surface species have reacted, the process (e.g., half-reaction) stops and additional reactant exposure produces no additional growth of the monolayer.

After the first half-reaction is completed, the reaction chamber can be purged and a second precursor species is introduced into the reaction chamber. The monolayer has reactive sites for reacting with the second precursor species. The second precursor species can be fed in vapor phase into the reaction chamber and can react with reactive sites on the surface of the previously formed monolayer. The chemisorptions of the second precursor proceeds until all reactive sites on the monolayer have reacted, at which point the reaction self-terminates, resulting in another monolayer which has reactive sites capable of reacting with the first precursor. The reaction chamber can be purged. Additional first and second precursor monolayers can be deposited by additional reaction cycles until the desired coating thickness is achieved.

In one example, a ceramic layer 126 maybe formed by the following two sequential half-reactions:

:Al-OH+Al(CH₃)_{3(g)}→:Al-O-Al(CH₃)₂+CH₄(g)

:Al-O-Al(CH₃)₂+2H₂O(g)→:Al-O-AL(OH)₂+2CH₄(g)

In one example, each half-reaction contributes around 0.1 nm thickness and the repeated switching between the two pre-cursors provides control over the thickness of the coating.

Atomic layer deposition processes can provide pinhole free layers or coatings. Atomic layer deposition processes can also provide a layer having more uniformity in thickness and/or a higher degree of conformity. The nanolaminate 124 and processes of forming the nanolaminate 124 described herein may be useful in coating medical devices having a spatially challenging structure where deposition or coating uniformity may otherwise be difficult to achieve, such as on the inner surface of tubing and conduits. The nanolaminate 124 and processes of forming the nanolaminate 124 described herein may also be useful for coating medical devices having very high aspect ratios (such as deep and narrow trenches).

Alternative methods for forming the ceramic layers 126 include sol gel processes, liquid-source misted chemical deposition (LSMCD), and pulsed plasma-enhanced chemical vapor deposition and radiation-induced vapor deposition. In LSMCD, a liquid precursor is delivered to a substrate as a fine mist which coalesces on the substrate surface. The substrate is subsequently subjected to a thermal process during which the solvent of the precursor evaporates, leaving a thin layer of solid on the substrate surface.

The polymer layers 128 may also be formed by processes enabling control over the thickness of the layers. Suitable processes include molecular layer deposition, sol gel processes, LSMCD, and plasma-enhanced chemical vapor deposition (PECVD). Although nanolaminate 124 has been described for use on a lead body, it will be recognized that nanolaminate 124 can be used on other medical devices. Suitable medical devices may have a tubular shape, such as a lead body or a catheter, or may have another shape.

FIG. 3 is a cross-sectional view of a lead body 210. One or more lead wires 230 extend through a lumen 222. A nanolaminate 224 can be on the outer surface of the lead wires 230 and can comprise ceramic layers 226a and 226b (referred to collectively as the ceramic layers 226) and a polymer layer 228. As shown, in some embodiments, the ceramic layers 226a and 226b and the polymer layer 228 can be arranged in an alternating pattern, and other patterns are also possible.

The nanolaminate 224 provides the lead wires 230 with an ultrathin but robust insulation. The ceramic layers 226 include a ceramic material, such as aluminum oxide, titanium oxide, bismuth oxide, vanadium oxide, hafnium oxide, zirconium oxide, silicone oxide, and zinc oxide, among others. As discussed herein, the lead wires 230 can include multiple layers of the ceramic layers 226, for example, the ceramic layers 226a and 226b. Each discrete ceramic layer 226 may have the same composition. In other embodiments, the ceramic layers 226 can have different compositions.

As described herein with respect to the ceramic layers 126, the ceramic layers 226 may be formed by a self-limiting reaction process which provides control over the thickness of the ceramic layers 226. In one example, the ceramic layers 226 are formed by atomic layer deposition (ALD). Each ceramic layer 226 may have a thickness less than or equal to 30 nm. As described herein ceramic layers 226 having a thickness less than or equal to 30 nm have a reduced risk for cracking or delamination compared to ceramic layers 226 having a thickness greater than 30nm.

The nanolaminate 224 insulates lead wires 230. For low voltage leads, such as up to about 20 volts and more particularly up to about 10 volts, a single 5 nm thick ceramic layer 226 may provide sufficient insulation. For higher voltage wires, a thicker ceramic layer 226 is necessary. While a polymer, such as a polyimide, polyamide or Teflon, may be used to provide sufficient insulation, these materials do not provide as high of wear resistance as a ceramic material. The nanolaminate 224 provides sufficient insulation to protect surrounding tissues as well has an enhanced wear resistance at least in part because the nanolaminate 224 includes multiple ceramic layers 226 separated by polymer layers 228.

As described herein, the nanolaminate 224 has sufficient insulating properties to protect tissue surrounding lead body 210 from the lead wires 230. Breakdown voltage is the minimum voltage required to cause an insulating material to become electrically conductive. The overall breakdown voltage of a nanolaminate 224 can be modeled by calculating the individual electric field over each individual layer. In one example, each layer of the nanolaminate 224 has a breakdown voltage greater than about 4 megavolts per centimeter (MV/cm). In another example, each layer of the nanolaminate 224 can have a breakdown voltage between about 4 MV/cm and about 12 MV/cm.

The nanolaminate 224 may be in addition to or as an alternative to nanolaminate 124 of FIG. 2. As described herein, each ceramic layer 226 may be equal to or less than 30 nm thick. Additionally, the polymer layers 228 may be positioned between the ceramic layers 226. In some embodiments, the polymer of the polymer layers 228 can intertwine with the ceramic layers 226 at the interface of the layers. The nanolaminate 224, the ceramic layers 226 and the polymer layer 228 may have the same composition and may be formed by the same processes as described herein for nanolaminate 124.

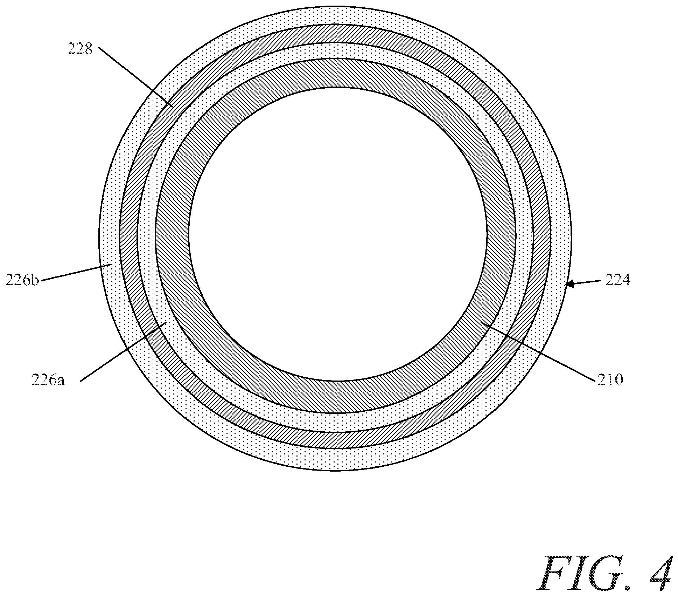

FIG. 4 is a cross-sectional view of a lead body 210 having a nanolaminate 224 applied to the outer surface. When applied to the outer surface of the lead body 210, the nanolaminate 224 may provide enhanced wear resistance as well as enhanced slip characteristics. For example, the nanolaminate 224 may have a lower coefficient of friction and a higher wear resistance than the outer surface of the lead body 210. In a still further example, the nanolaminate 224 may be applied to the outer surface of a catheter. The nanolaminate 224 may have a lower coefficient of friction and a higher wear resistance than the outer surface of the catheter such that the nanolaminate 224 provides enhanced wear resistance and enhanced slip characteristics.

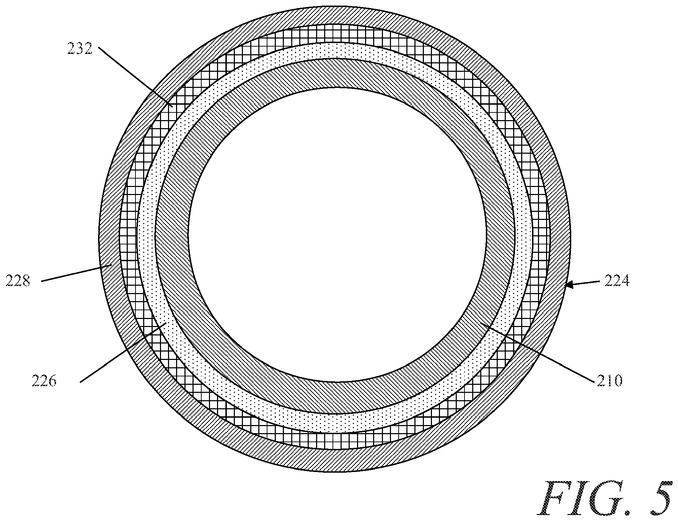

FIG. 5 is a cross-sectional view of a lead body 210 having a nanolaminate 224 including a ceramic layer 226, a polymer layer 228 and a hybrid layer 232. The hybrid layer 232 can be positioned between the ceramic layer 226 and the polymer layer 228. The hybrid layer 232 can include ceramic material and polymer material. For example, the hybrid layer 232 may be produced by combining inorganic and organic reactants. Additionally, the hybrid layer 232 may be formed by a self-limiting process, which enables the thickness of the hybrid layer 232 to be controlled.

For example, trimethyaluminum (TMA) and ethylene glycol (EG) can be combined to produce a product known as alucone. In this reaction scheme, the ratio of ceramic to polymer material can be changed by substituting water for a portion of the ethylene glycol. For example, where EG is represented by A, TMA is represented by B and water is represented by C, a suitable assembly sequence can be A, B, A, B, C, B, A, B, C, B, and so on. This hybrid layer 232 has mechanical properties between those of the pure polymer (ethylene glycol) and the pure ceramic material (TMA). As shown in FIG. 5, the nanolaminate 224 on the outer surface of the lead body 210 can include the hybrid layer 232. Nanolaminates on the inner surface of lead bodies, on lead wires or on the outer surface of a catheter, among other medical devices, may also include hybrid layer 232.

The hybrid layer 232 may have thicknesses less than 30 nm to reduce the risk of cracking or delamination. For example, the hybrid layer 232 may have a thickness from about 5 nm to about 30 nm. In one example, the hybrid layer 232 can be used between a ceramic layer 226 and a polymer layer 228 of the nanolaminate 224. The hybrid layer 232 may intertwine discrete ceramic and polymer layers 226 and 228, respectively, and may smooth the transition between the layers. It will be recognized that a hybrid layer 232 can be formed with alternative reactants. For example, diethylzinc may be used in place of TMA.

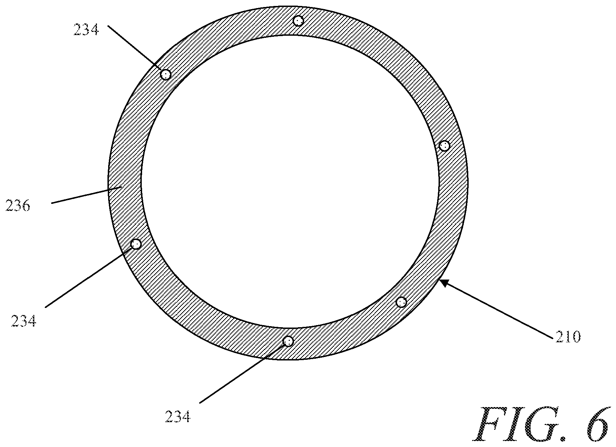

FIG. 6 is a cross-sectional view of a lead body 210 which includes a ceramic material 234 dispersed throughout a polymer material 236. As discussed herein, a ceramic material may have higher wear resistance and increased strength compared to polymer materials. The ceramic material 234 may be dispersed throughout the polymer material 236 to provide enhanced strength and/or wear resistance. For example, the ceramic material 234 may be homogenously dispersed throughout the polymer material 236.

In one example, the polymer material 236 is formed by first coating or depositing ceramic material onto beads or particles of the polymer material. The coated polymer beads are then extruded to form the medical device, such as a lead body. Extrusion of the coated beads results in the ceramic material 234 being dispersed throughout the extruded polymer material 236.

The ceramic material 234 may be deposited by a self-limiting reaction to provide control over the thickness of the ceramic material coating formed on the polymer beads. For example, a ceramic coating may be formed by ALD, as described herein.

In some embodiments, nanoclay and/or nanodiamond material may be incorporated into the polymer material 236 in addition to or in place of the ceramic material 234.

The nanoclay material can improve the strength of the polymer material 236 and provide increased wear resistance. Nanoclay materials can have a high aspect ratio. For example the nanoclay material may have a plate-like structure (also referred to as platey or platy structure) in which the dimensions in two-dimensions far exceed the structure's thickness Plate-like structure nanoclays can have an unusually high aspect ratio. For example, individual platelet thicknesses can be about 1 nm and the surface dimensions can be at least about 300 nm to more than about 600 nm. Suitable nanoclay materials include but are not limited to nanoclays available from Southern Clay Products, Inc, Gonzales, TX. Suitable nanoclay materials may be montmorillonite clays that are reacted with quaternary ammonium to allow dispersion. In one example, Cloisite® and Claytone® are useful additives.

The nanoclay material may be incorporated into the polymer material prior to extrusion. In one example, the nanoclay material is compounded with the polymer material by a twin screw extruder to form a mixed material. The mixed material can be chopped into pellets within the twin screw extruder and the mixed material can be formed into a desired product, such as a lead body, in a single screw extruder. After extrusion, the nanoclay material is dispersed in the extruded polymer material. Extruding polymer beads containing the nanoclay material may produce an extruded polymer material having the nanoclay homogenous dispersed therein. More specifically, the nanoclay material can be homogenously dispersed in three-dimensions (length, width and thickness) throughout the polymer material. Following extrusion, a ceramic coating may be applied to the polymer material by ALD or another process as described herein.

In some embodiments, nanodiamond materials may be used in combination with or as an alternative to ceramic material and the nanoclay material. Nanodiamond materials can improve the strength of the polymer material and provide increased wear resistance. Nanodiamonds are nanosized diamond particles having a crystal size between about 1 nm and about 100 nm, less than about 50 nm, or less than about 20 nm. Suitable nanodiamond materials include but are not limited to uDiamond® Molto available from Carbodeon, Vantaa, Finland.

Nanodiamond materials can be mixed with the polymer material prior to extrusion as discussed above with respect to the nanoclay. Similar to the nanoclay materials, following extrusion, the nanodiamond materials can be homogenously dispersed throughout the polymer material.

A medical device, such as a lead body, can be formed of the polymer material 236 containing ceramic material 234, nanoclay material, nanodiamond material or a combination thereof. As discussed herein, ceramic material 234, nanoclay material and nanodiamond material can increase the strength and wear resistance of the polymer material. A lead body 210 including a polymer material 236 containing ceramic material 234, nanoclay material, nanodiamond material or a combination thereof can have improved wear resistance and a reduced risk of isolation failure.

Various modifications and additions can be made to the exemplary embodiments discussed without departing from the scope of the present invention. For example, while the embodiments described above refer to particular features, the scope of this invention also includes embodiments having different combinations of features and embodiments that do not include all of the described features. Accordingly, the scope of the present invention is intended to embrace all such alternatives, modifications, and variations as fall within the scope of the claims, together with all equivalents thereof.

## Claims

1. An implantable medical lead (102) comprising:
a substrate having a surface; and
a nanolaminate (124; 224) disposed on the surface of the substrate, the nanolaminate (124; 224) comprising:
at least one ceramic layer (126; 226); and
at least one polymer layer (128; 228), wherein each ceramic layer (126; 226) is less than or equal to about 30 nanometers thick.

2. The implantable medical lead (102) of claim 1 wherein a polymer layer (128; 228) is disposed between a first ceramic layer (126a; 226a) and a second ceramic layer (126b; 226b).

3. The implantable medical lead (102) of claim 1 or 2, wherein the ceramic layers (126; 226) and the polymer layers (128; 228) are arranged in an alternating pattern.

4. The implantable medical lead (102) of any of claims 1-3, wherein the polymer layers (128; 228) are less than about 100 nanometers thick.

5. The implantable medical lead (102) of any of claims 1-4, wherein the ceramic layer (126; 226) includes at least one component selected from the group consisting of aluminum oxide and titanium oxide.

6. The implantable medical lead (102) of any of claims 1-5, wherein the implantable medical lead (102) comprises a tubular body (110; 210) having an outer surface and an inner surface, and wherein the nanolaminate (124; 224) is disposed on the outer surface.

7. The implantable medical lead (102) of any of claims 1-6, wherein the implantable medical lead (102) comprises a tubular body (110; 210) having an outer surface and an inner surface, and wherein the nanolaminate (124; 224) is disposed on the inner surface.

8. The implantable medical lead (102) of any of claims 1-7, and further comprising at least one layer (232) comprising a ceramic material and a polymer material.

9. A method of forming a nanolaminate (124; 224) on an implantable medical lead (102), the method comprising:
depositing a ceramic material on the implantable medical lead (102) to form a ceramic layer (126a; 226a) having a thickness less than or equal to about 30 nanometers; and
depositing a polymer material on the ceramic layer to form a polymer layer (128; 228).

10. The method of claim 9 and further comprising depositing a second ceramic material on the polymer layer (128; 228) to form a second ceramic layer (126b; 226b).

## Patentansprüche

1. Implantierbare medizinische Leitung (102), welche aufweist:
ein Substrat mit einer Oberfläche; und
ein Nanolaminat (124, 224), das auf der Oberfläche des Substrats angeordnet ist, welches Nanolaminat (124, 224) aufweist:
zumindest eine keramische Schicht (126, 226); und
zumindest eine Polymerschicht (128; 228), wobei jede Keramikschicht (126; 226) weniger als oder gleich etwa 30 Nanometer dick ist.

2. Implantierbare medizinische Leitung (102) nach Anspruch 1, bei der eine Polymerschicht (128; 228) zwischen einer ersten keramischen Schicht (126a; 226a) und einer zweiten keramischen Schicht (126b; 226b) angeordnet ist.

3. Implantierbare medizinische Leitung (102) nach Anspruch 1 oder 2, bei der die keramischen Schichten (126; 226) und die Polymerschichten (128; 228) in einem abwechselnden Muster angeordnet sind.

4. Implantierbare medizinische Leitung (102) nach einem der Ansprüche 1 - 3, bei der die Polymerschichten (128; 228) weniger als etwa 100 Nanometer dick sind.

5. Implantierbare medizinische Leitung (102) nach einem der Ansprüche 1 - 4, bei der die keramische Schicht (126; 226) zumindest eine Komponente enthält, die ausgewählt ist aus der Gruppe bestehend aus Aluminiumoxid und Titanoxid.

6. Implantierbare medizinische Leitung (102) nach einem der Ansprüche 1 - 5, bei der die implantierbare medizinische Leitung (102) einen rohrförmigen Körper (110; 210) mit einer äußeren Oberfläche und einer inneren Oberfläche aufweist, und bei der das Nanolaminat (124; 224) auf der äußeren Oberfläche angeordnet ist.

7. Implantierbare medizinische Leitung (102) nach einem der Ansprüche 1 - 6, bei der die implantierbare medizinische Leitung (102) einen rohrförmigen Körper (110; 210) mit einer äußeren Oberfläche und einer inneren Oberfläche aufweist, und bei der das Nanolaminat (124; 224) auf der inneren Oberfläche angeordnet ist.

8. Implantierbare medizinische Leitung (102) nach einem der Ansprüche 1 - 7, weiterhin aufweisend zumindest eine Schicht (232), die ein keramisches Material und ein Polymermaterial aufweist.

9. Verfahren zum Bilden eines Nanolaminats (124; 224) auf einer implantierbaren medizinischen Leitung (102), welches Verfahren aufweist:
Aufbringen eines keramischen Materials auf die implantierbare medizinische Leitung (102), um eine keramische Schicht (126a; 226a) mit einer Dicke von weniger als oder gleich etwa 30 Nanometern zu bilden; und
Aufbringen eines Polymermaterials auf die keramische Schicht, um eine Polymerschicht (128; 228) zu bilden.

10. Verfahren nach Anspruch 9, weiterhin aufweisend das Aufbringen eines zweiten keramischen Materials auf die Polymerschicht (128; 228), um eine zweite keramische Schicht (126b; 226b) zu bilden.

## Revendications

1. Sonde médicale implantable (102) comprenant :
un substrat qui comporte une surface ; et
un nano-stratifié (124 ; 224) qui est disposé sur la surface du substrat, le nano-stratifié (124 ; 224) comprenant :
au moins une couche en céramique (126 ; 226) ; et
au moins une couche en polymère (128 ; 128), dans laquelle chaque couche en céramique (126 ; 226) présente une épaisseur inférieure ou égale à environ 30 nanomètres.

2. Sonde médicale implantable (102) selon la revendication 1, dans laquelle une couche en polymère (128 ; 228) est disposée entre une première couche en céramique (126a ; 226a) et une seconde couche en céramique (126b ; 226b).

3. Sonde médicale implantable (102) selon la revendication 1 ou 2, dans laquelle les couches en céramique (126 ; 226) et les couches en polymère (128 ; 228) sont agencées selon un motif alterné.

4. Sonde médicale implantable (102) selon l'une quelconque des revendications 1 à 3, dans laquelle les couches en polymère (128 ; 228) présentent une épaisseur inférieure à environ 100 nanomètres.

5. Sonde médicale implantable (102) selon l'une quelconque des revendications 1 à 4, dans laquelle la couche en céramique (126 ; 226) inclut au moins un composant qui est sélectionné parmi le groupe qui est constitué par l'oxyde d'aluminium et l'oxyde de titane.

6. Sonde médicale implantable (102) selon l'une quelconque des revendications 1 à 5, dans laquelle la sonde médicale implantable (102) comprend un corps tubulaire (110 ; 210) qui comporte une surface externe et une surface interne, et dans laquelle le nano-stratifié (124 ; 224) est disposé sur la surface externe.

7. Sonde médicale implantable (102) selon l'une quelconque des revendications 1 à 6, dans laquelle la sonde médicale implantable (102) comprend un corps tubulaire (110 ; 210) qui comporte une surface externe et une surface interne, et dans laquelle le nano-stratifié (124 ; 224) est disposé sur la surface interne.

8. Sonde médicale implantable (102) selon l'une quelconque des revendications 1 à 7, et comprenant en outre au moins une couche (232) qui comprend un matériau de céramique et un matériau de polymère.

9. Procédé de formation d'un nano-stratifié (124; 224) sur une sonde médicale implantable (102), le procédé comprenant :
le dépôt d'un matériau de céramique sur la sonde médicale implantable (102) de manière à former une couche en céramique (126a ; 226a) qui présente une épaisseur inférieure ou égale à environ 30 nanomètres ; et
le dépôt d'un matériau de polymère sur la couche en céramique de manière à former une couche en polymère (128 ; 228).

10. Procédé selon la revendication 9, et comprenant en outre le dépôt d'un second matériau de céramique sur la couche en polymère (128 ; 228) de manière à former une seconde couche en céramique (126b ; 226b).
